(19) Europäisches Patentamt
European Patent Office
Office européen des brevets

(11) EP 1 176 136 A1

# (12) EUROPEAN PATENT APPLICATION
published in accordance with Art. 158(3) EPC

(43) Date of publication:
**30.01.2002 Bulletin 2002/05**

(21) Application number: **01904427.0**

(22) Date of filing: **14.02.2001**

(51) Int Cl.[7]: **C07C 67/333**, C07C 69/60, C08G 18/83, C08G 63/91 // (C07B61/00, B01J31:24, 23:46, 27:13)

(86) International application number:
**PCT/JP01/01028**

(87) International publication number:
**WO 01/60780 (23.08.2001 Gazette 2001/34)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **15.02.2000 JP 2000041343**

(71) Applicant: **SHOWA DENKO K.K.**
**Tokyo 105-8518 (JP)**

(72) Inventors:
- **KADOWAKI, Yasushi**
  **Oita Plant Petrochemicals Sector**
  **Oita-shi Oita 870-0189 (JP)**
- **OHTA, Keisuke**
  **Oita Plant Petrochemicals Sector**
  **Oita-shi Oita 870-0189 (JP)**
- **KAI, Kazufumi Oita Plant Petrochemicals Sector**
  **Oita-shi Oita 870-0189 (JP)**

(74) Representative: **Strehl Schübel-Hopf & Partner**
**Maximilianstrasse 54**
**80538 München (DE)**

## (54) PROCESS FOR PRODUCING FUMARIC ESTER

(57) A method for isomerizing a maleate, or a maleate containing a 2-propenyloxy group in the molecule at a high selectivity is provided which employs an isomerizing catalyst containing a Group VIII element. This method produces a fumarate, or a fumarate containing a 1-propenyloxy group in a high yield, which is useful in the fields of resin source materials and plasticizer. This method gives high-purity fumarates containing no catalyst residue by use of a heterogeneous catalyst which is readily separable and non-corrosive.

EP 1 176 136 A1

## Description

Field of the Invention

The present invention relates to a process for producing a fumarate by isomerization of a maleate. The present invention relates also to a process for producing a fumarate containing a 1-propenyloxy group by isomerizing a maleate containing a 2-propenyloxy group in the molecule through isomerization of the 2-propenyloxy group to the 1-propenyloxy group and concurrent isomerization of the maleate to a fumarate. The produced fumarate is highly polymerizable and capable of forming a polymer having excellent properties, being suitable for resin applications such as paints and adhesives.

Related Background Art

The double bond of a fumaric acid residue is known to be readily polymerizable in comparison with the double bond of a maleic acid residue, and is highly copolymerizable with a reactive diluent like styrene. For example, in production of an unsaturated polyester resin, maleic anhydride is widely used as a polybasic acid anhydride source. In production of a resin with this material, the maleate is known to isomerize to the fumarate depending on the combinedly used saturated polybasic acid and polyhydric alcohol, and the production temperature. However, the isomerization is not always complete in the produced unsaturated polyester depending on the production conditions. To solve such problems, a process for producing a fumarate is disclosed in which a maleate is isomerized to a fumarate in the presence of an acid chloride (Japanese Patent Application Laid-Open No. 9-77861). However, in this process, the stainless-steel reactor and stirrer for the reaction are liable to be corroded. Further, this process is applicable only to unsaturated polyester resin production, and is not generally applicable, for example, to isomerization of the maleic acid skeleton having functional groups to the fumaric acid skeleton.

The known processes therefor include isomerization by a base such as piperidine, morpholine, and diethylamine (J.Polym.Sci. Part A Polym.Chem. (1992), 30(7), 1347); and isomerization by an acid such as concentrated hydrochloric acid, and concentrated sulfuric acid. However, in the isomerization by the base, the catalyst cannot readily be removed completely, giving adverse effect in curing. In the isomerization by the acid, the stainless-steel reactor and stirrer for the reaction may be corroded and the remaining chloride ions or sulfate ions can affect adversely in curing.

In a still another known process, the isomerization of a maleate to a fumarate is conducted by use of a homogeneous catalyst (Japanese Patent Publication No. 50-17044). This process, however, requires a high temperature of 150°C or higher, a high pressure of 50 atm or higher, and use of carbon monoxide, hydrogen, or the like which is a toxic gas or an explosive gas, and produces a large amount of byproducts by hydroformylation or hydrogenation, disadvantageously.

Recently, radical-polymerizable monomers having a 1-propenyloxy group and an unsaturated dibasic acid residue like fumaric acid residue are reported to be useful (WO9902482). Such a monomer is highly polymerizable, but not irritating the skin, and being affected less by oxygen in polymerization, advantageously.

In one possible method for producing such a polymerizable monomer, a monomer is synthesized which contains a 2-propenyloxy group and a maleic acid residue, and then the 2-propenyloxy group is isomerized to a 1-propenyloxy group and separately the maleic acid residue is isomerized to a fumaric acid residue, in two-step isomerization. In another possible method, the both groups are isomerized in one step. The method of isomerizing the respective groups in separate steps is complicated owing to the two-step reaction to cause a drop of the overall yield due to the losses in the respective steps. Therefore, desirably, the respective isomerization steps are conducted simultaneously in one step under mild conditions, if possible.

DISCLOSURE OF THE INVENTION

The present invention was made under the above circumstances. An object of the present invention is to provide a process for producing a fumarate at a low cost by isomerization of a maleate with a highly active and less corrosive catalyst under mild reaction conditions.

Another object of the present invention is to provide a simpler method for producing a fumarate in a high purity from a maleate by use of a heterogeneous catalyst which is readily separable after the reaction and is less corrosive.

A further object of the present invention is to provide a process for producing a fumarate containing a 1-propenyloxy group by isomerization of a maleate containing a 2-propenyloxy group in the molecule by isomerizing the 2-propenyloxy group to the 1-propenyloxy group and concurrently isomerizing the maleate to the fumarate in one step.

The inventors of the present invention, after comprehensive investigation, have found a method for producing fumarates industrially at a high selectivity in a high yield through isomerization of a maleate by use of a catalyst containing a Group VIII element.

Further, the inventors of the present invention have found that fumarates can be produced industrially at a high selectivity in a high yield without a remaining catalyst through steps of isomerizing a maleate in the presence of a supported Group VIII metal as a catalyst and removing the catalyst by filtration or a like operation. Thus the present invention has been completed. The present invention is summarized as below.

(1) A method for producing a fumarate by isomerizing a maleate, comprising using an isomerization catalyst containing a Group VIII element.
(2) The method for producing a fumarate according to the above invention (1), wherein the isomerization catalyst is a compound containing a Group VIII element.
(3) The method for producing a fumarate according to the above invention (2), wherein the isomerization catalyst is a metal salt or a metal complex containing at least one element selected from the group consisting of ruthenium, rhodium, palladium, iridium, and platinum.
(4) The method for producing a fumarate according to the above invention (3), wherein the isomerization catalyst is a ruthenium salt, a ruthenium complex, a rhodium salt, or a rhodium complex.
(5) The method for producing a fumarate according to the above invention (4), wherein the isomerization catalyst is $RuCl_2(PPh_3)_3$, $RhCl(CO)(PPh_3)_2$, or $RuClH(CO)(PPh_3)_3$.
(6) The method for producing a fumarate according to the above invention (2), wherein a phosphine compound represented by a chemical formula $PR_3$ (R denoting independently an alkyl, a cycloalkyl, or a phenyl), and/or a base is employed in addition to the above isomerization catalyst.
(7) The method for producing a fumarate according to the above invention (6), wherein the amount of the added phosphine compound ranges from 0.01 to 50.00 parts by weight based on 100 parts by weight of the maleate, and/or the amount of the added base ranges from 0.01 to 50.00 parts by weight based on 100 parts by weight of the maleate.
(8) The method for producing a fumarate according to the above invention (1), wherein the isomerization catalyst is one or more kinds of supported catalyst in which the Group VIII metal is supported on a carrier in an amount ranging from 0.05% to 10% by weight based on the entire weight of the catalyst.
(9) The method for producing a fumarate according to the above invention (8), wherein the Group VIII metal is at least one metal selected from the group consisting of ruthenium, rhodium, palladium, iridium, and platinum.
(10) The method for producing a fumarate according to the above invention (9), wherein at least one Group VIII metal of the isomerization catalyst is ruthenium.
(11) The method for producing a fumarate according to the above invention (10), wherein one or more supported catalysts are used in which the ruthenium metal is supported on the carrier in an amounts ranging from 0.05% to 10% by weight based on the entire weight of the catalyst.
(12) A method for producing a fumarate containing a 1-propenyloxy group, comprising isomerizing a maleate containing a 2-propenyloxy group in the molecule thereof to the fumarate containing the 1-propenyloxy group by use of an isomerization catalyst containing a Group VIII element.
(13) The method for producing a fumarate containing a 1-propenyloxy group according to the above invention (12), wherein the isomerization catalyst is a compound containing a Group VIII element.
(14) The method for producing a fumarate containing a 1-propenyloxy group according to the above invention (13), wherein the isomerization catalyst is a metal salt or a metal complex containing at least one element selected from the group consisting of ruthenium, rhodium, palladium, iridium, and platinum.
(15) The method for producing a fumarate containing a 1-propenyloxy group according to the above invention (14), wherein the isomerization catalyst is a ruthenium salt, a ruthenium complex, a rhodium salt, or a rhodium complex.
(16) The method for producing a fumarate containing a 1-propenyloxy group according to the above invention (15), wherein the isomerization catalyst is $RuCl_2(PPh_3)_3$, $RhCl(CO)(PPh_3)_2$, or $RuClH(CO)(PPh_3)_3$.
(17) The method for producing a fumarate containing a 1-propenyloxy group according to the above invention (13), wherein a phosphine compound represented by a chemical formula $PR_3$ (R denoting independently an alkyl, a cycloalkyl, or a phenyl), and or a base is employed in addition to the above isomerization catalyst.
(18) The method for producing a fumarate containing a 1-propenyloxy group according to the above invention (17), wherein the amount of the added phosphine compound ranges from 0.01 to 50.00 parts by weight based on 100 parts by weight of the maleate, and/or the amount of the added base ranges from 0.01 to 50.00 parts by weight based on 100 parts by weight of the maleate.
(19) The method for producing a fumarate containing a 1-propenyloxy group according to the above invention (12), wherein the isomerization catalyst is one or more kinds of supported catalyst in which a Group VIII metal is supported on a carrier in an amount ranging from 0.05% to 10% by weight based on the entire weight of the catalyst.
(20) The method for producing a fumarate containing a 1-propenyloxy group according to the above invention (19), wherein the Group VIII metal is at least one metal selected from the group consisting of ruthenium, rhodium, palladium, iridium, and platinum.

(21) The method for producing a fumarate containing a 1-propenyloxy group according to the above invention (20), wherein at least one Group VIII metal of the isomerization catalyst is ruthenium.

(22) The method for producing a fumarate containing a 1-propenyloxy group according to the above invention (21), wherein one or more supported catalysts are used in which the ruthenium metal is supported on the carrier in an amount ranging from 0.05% to 10% by weight based on the entire weight of the catalyst.

DETAILED DESCRIPTION OF THE PREFERRED EMBODIMENT

The present invention is described below in detail.

The numbers for referring to the invention correspond to the reference numbers of the invention in the above description of "Disclosure of the Invention".

The maleate employed in the present invention includes compounds having a structure unit represented by General Formula (I) below, having a double bond of a maleic acid residue,

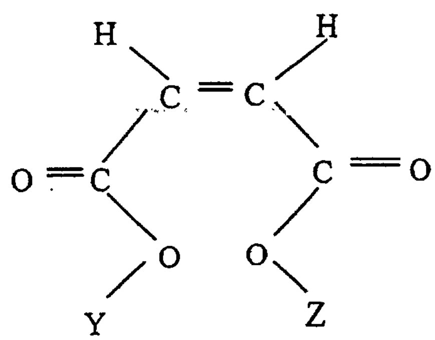

... (I)

where Y and Z are independently H or an organic group respectively, but Y and Z are not simultaneously H.

The compound may have one or more of this structure unit in one and the same molecule. That is, the maleate includes compounds having two or more double bonds of the maleic acid residue in one and the same molecule. The maleate compounds include dialkyl maleates such as dimethyl maleate, diethyl maleate, dipropyl maleate, diisopropyl maleate, and dibutyl maleate; dialkenyl maleates such as diallyl maleate; bis(alkenyloxyalkyl) maleates such as bis (2-(2-propenyloxy)ethyl) maleate, bis(3-(2-propenyloxy)propyl) maleate, and bis(4-(2-propenyloxy)butyl) maleate; alkyl hydrogen maleates such as methyl hydrogen maleate, ethyl hydrogen maleate, propyl hydrogen maleate, isopropyl hydrogen maleate, and butyl hydrogen maleate; alkenyl hydrogen maleate such as allyl hydrogen maleate; esters such as propoxyethyl hydrogen maleate, dipropoxyethyl maleate, monoallyloxyethyl maleate, and diallyloxyethyl maleate; and alkenyloxyalkyl maleates such as 2-(2-propenyloxy)ethyl maleate, 3-(2-propenyloxy)propyl maleate, and 4-(2-propenyloxy)butyl maleate. The maleate compounds include also polymers and oligomers such as unsaturated polyesters and urethane oligomers containing maleate units. The maleates which are applicable in the present invention are not limited thereto.

The present invention provides, as mentioned above, a method for producing a fumarate by isomerizing a maleate in the presence of a catalyst to a corresponding fumarate. Further, the present invention provides a method for producing a fumarate containing a 1-propenyloxy group by isomerizing the 2-propenyloxy group to 1-propenyloxy group as shown by the formula below and concurrently isomerizing the maleate to a fumarate to produce a fumarate containing a 1-propenyloxy group in the presence of a catalyst. The maleate containing a 2-propenyloxy group includes bis(alkenyloxyalkyl) maleates such as bis(2-(2-propenyloxy)ethyl) maleate, bis(3-(2-propenyloxy)propyl) maleate, and bis(4-(2-propenyloxy)butyl) maleate; alkenyloxyalkyl maleates such as 2-(2-propenyloxy)ethyl maleate, 3-(2-propenyloxy)propyl maleate, and 4-(2-propenyloxy)butyl maleate; and unsaturated polyesters, other polymers or oligomers containing maleate units containing a 2-propenyloxy group.

$$CH_2{=}CH{-}CH_2{-}O{-} \rightarrow CH_3{-}CH{=}CH_2{-}O{-} \qquad (II)$$

In this specification of the present invention, the "maleate" includes the above mentioned maleates and derivatives thereof, and polymers and oligomers such as unsaturated polyesters and urethane oligomers containing the

maleate units; and the "fumarate" includes the above mentioned fumarates and derivatives thereof, and polymers and oligomers such as unsaturated polyesters and urethane oligomers containing the fumarate units.

The present invention is described below in more detail.

The present invention (1) relates to a method for producing a fumarate by isomerizing a maleate by use of an isomerization catalyst containing a Group VIII element. The "isomerization catalyst" herein is employed in the reaction of isomerization of a maleate to a fumarate, and in the reaction of isomerization of a maleate containing a 2-propenyloxy group in the molecule; the isomerization catalyst catalyzes isomerization of 2-propenyloxy group to a 1-propenyloxy group and simultaneously catalyzes isomerization of a maleate to a fumarate. The catalyst includes compounds containing a Group VIII element, and also one or more supported catalysts in which or a metal of Group VIII is supported on a carrier in an amount ranging from 0.05% to 10% by weight based on the entire weight of the catalyst, as shown below.

The present invention (2) relates to a method for producing a fumarate by isomerizing a maleate by use of a compound containing a Group VIII element as the catalyst.

The type of the compound containing the Group VIII element for the isomerization reaction includes metals; inorganic salts such as sulfate, nitrate, and chlorides; organic acid salts such as acetates, and oxalates; and a metal complexes having a coordinated ligand. Of these types of compounds, particularly preferred are metal complexes having a suitable ligand.

The ligand of the metal complex includes triphenylphosphine, carbonyl, hydrido, halogeno, aqua, cyclopentadiene, cyclooctadiene, acetylacetonato, and allyl ligands, but is not limited thereto.

The metal complex includes
dichlorotris(triphenyl)phosphineruthenium (II),
dichlorotetrakis(triphenyl)phosphineruthenium (II), trirutheniumdodecacarbonyl,
carbonylchlorohydridotris(triphenyl)phosphineruthenium (II), chlorocyclopentadienylbis(triphenyl)phosphineruthenium (II), bis(cyclopentadienyl)ruthenium,
carbonyldihydridotris(triphenyl)phosphineruthenium (II), dicarbonyldichlorobis(triphenyl)phosphineruthenium (II), chlorocarbonylbis(triphenyl)phosphinerhodium (I),
tetrakis(triphenyl)phosphinepalladium (0),
bis(acetato)bis(triphenyl)phosphinepalladium (II), and dicarbonylcyclopentadienyliron dimer, but is not limited thereto.

The metal complex, as the catalyst, may be used as it is without modification. The metal complex may be formed during the reaction by adding the metal complex components, namely the metal, the ligand, and other component, separately to the reaction material to form the metal complex during the reaction. Otherwise, a compound formed by decomposition of the metal complex may be used as the catalyst.

The amount of the isomerization catalyst employed in the invention (2) ranges preferably from 0.01 to 50.00 parts, more preferably from 0.10 to 30.00 parts by weight based on 100 parts by weight of the maleate. With the catalyst in an amount of less than 0.01 parts by weight, the isomerization reaction rate is lower, requiring a longer time for achieving a high isomerization ratio, which is disadvantagaeous industrially. With the catalyst in an amount of more than 50.00 parts by weight, the catalyst cost is higher, which is disadvantageous economically, even though the reaction rate is higher.

The catalyst in the present invention may be used singly or in combination of two or more thereof.

The present inventions (3) to (5) are described in detail below. In the inventions (3) to (5), as the catalyst, a metal salt or a metal complex containing at least one of elements of ruthenium, rhodium, palladium, iridium, and platinum is employed selectively from the compounds containing Group VIII element employed in the invention (2).

It was found by the inventors of the present invention that, in the isomerization reaction of the maleate by use of the compound containing a Group VIII element shown in the invention (2), the compound containing iron, cobalt, or nickel tends to exhibit relatively low activity among the Group VIII elements, although the reason is not clear. The elements like iron, cobalt, and nickel are relatively inexpensive and are readily available industrially. However, the amount of the catalyst should be increased owing to low catalytic activity.

On the other hand, the metal salt or the metal complex containing at least one element selected from the group of ruthenium, rhodium, palladium, iridium, and platinum of the same Group VIII was found to give higher activity and produces less byproducts with suitable selection of the ligand. Of the compounds containing ruthenium, rhodium, palladium, iridium, or platinum, particularly preferred are compounds containing ruthenium or rhodium: ruthenium salts, ruthenium complexes, rhodium salts, and rhodium complexes. Of these, the most suitable are $RuCl_2(PPh_3)_3$, $RhCl(CO)(PPh_3)_2$, and $RuClH(CO)(PPh_3)_3$.

In industrial production of a fumarate, the use of the latter elements (ruthenium, rhodium, palladium, iridium, and platinum) is more economical in consideration of the overall production process, although the reason therefor is not clear.

The present invention (6) is described below in detail. The invention (6) relates to a method for producing a fumarate, wherein a phosphine compound represented by the chemical formula $PR_3$ and/or a base is employed in

addition to the aforementioned isomerization catalyst in the isomerization reaction of a maleate. The symbol R denotes a group of an alkyl, a cycloalkyl, or a phenyl, each R being the same or different.

As shown in the inventions (2) to (5), the inventors of the present invention found the method for producing a fumarate by isomerization of a maleate by use of a compound containing a Group VIII element as the catalyst.

Furthermore, the inventors of the present invention found that use of a phosphine compound represented by the chemical formula $PR_3$ and/or a base in addition to the isomerization catalyst increases the isomerization rate and raises the selectivity dramatically. The high catalytic activity enables decrease of the amount of the catalyst and decrease of the reaction time, resulting in reduction of the production cost desirably in industrial production.

The catalyst employed in the invention (6) may be the same compound containing Group VIII element as that employed in the invention (2).

The type of the compound containing the Group VIII element in the invention (6) is the same as that in the invention (2), including metals, inorganic salts, organic salts, and metal complexes having various ligands. Of these, particularly preferred are metal complexes having a suitable ligand.

The ligand of the metal complex includes triphenylphosphine, carbonyl, hydrido, halogeno, aqua, cyclopentadiene, cyclooctadiene, acetylacetonato, and allyl ligands, but is not limited thereto. The usage of such a metal complex is the same as that described in the invention (2).

The amount of the isomerization catalyst employed in the invention (6), similarly as in the invention (2), ranges preferably from 0.01 to 50.00 parts, more preferably from 0.10 to 30.00 parts by weight based on 100 parts by weight of the maleate. With the catalyst in an amount of less than 0.01 parts by weight, the isomerization reaction rate is lower, requiring a longer time for achieving a high isomerization ratio, which is disadvantagaeous industrially. With the catalyst in an amount of more than 50.00 parts by weight, the catalyst cost is higher, which is disadvantageous economically, although the reaction rate is higher.

The additive employed in the invention (6) is a phosphine compound represented by a chemical formula $PR_3$ and/or a base. The additive is explained below in detail.

The substituent R of the phosphine compound $PR_3$ includes usual substitutents such as alkyl, cycloalkyl, and phenyl. Specific examples are tri-n-butylphosphine, triphenylphosphine, and tricyclohexylphosphine, but are not limited thereto. Each of the R substitutents may be the same or different.

On the other hand, the base employed as the additive includes usual inorganic bases and organic bases. The inorganic base includes sodium hydroxide, potassium hydroxide, calcium hydroxide, and ammonia. The organic base includes triethylamine, tripropylamine, pyridine, and morpholine. However, the base is not limited thereto.

The cause of the effect of the additive has not been elucidated. The addition of a phosphine compound is effective mainly in promoting the isomerization of a maleic acid residue to a fumaric acid residue, whereas the addition of a base is effective mainly in promoting the isomerization of the 2-propenyloxy group to the 1-propenyloxy group. Addition of either one compound is effective to lower the selectivity of an undesirable byproduct polymer. The decrease of the formation of the polymer increases the monomer yield advantageously.

The present invention (7) is explained below in detail. The invention (7) shows the optimum range of addition of the phosphine compound and/or the base. As shown in the invention (6), the inventors of the present invention found that use of a phosphine compound represented by the chemical formula $PR_3$ and/or a base in addition to the isomerization catalyst increases the isomerization rate and raises the selectivity dramatically.

The mechanism of the above action has not been elucidated as yet. The additive itself causes little the isomerization reaction, but a combination of the additive with the isomerization catalyst promotes remarkably the reaction. Therefore, the effect of the addition is presumed to be due to an interaction or synergism between the isomerization catalyst and the additive.

With the phosphine added in an amount of as small as 0.01 part by weight based on 100 parts by weight of the maleate, and/or with the base added in an amount of as small as 0.01 part by weight based on 100 parts by weight of the maleate, the additive would not interact sufficiently with the isomerization catalyst, exhibiting little effect of addition.

On the other hand, with the phosphine added in a larger amount of more than 50.00 parts by weight based on 100 parts by weight of the maleate, and/or with the base added in a larger amount of more than 50.00 parts by weight based on 100 parts by weight of the maleate, the effect of the addition is saturated and the reaction rate does not increase more. Therefore such a larger amount of the addition is not effective.

Furthermore, use of a larger amount of the additive results in an increase of the production cost economically disadvantageous. Therefore, there is an optimum range of the amount of the additive.

The present inventions (8) to (11) show the use of one or more catalysts of a Group VIII metal supported on a carrier in the isomerization. The amount of the supported Group VIII metal ranges preferably from 0.05% to 10% by weight based on the total weight of the metal-supporting catalyst (carrier and Group VIII metal).

The present invention (9) shows that ruthenium, rhodium, palladium, iridium, and platinum are preferred among the Group VIII metals in view of the activity.

The present invention (10) shows that ruthenium is particularly preferred as at least one of the Group VIII metals as the isomerization catalyst.

The present invention (11) shows that one or more supported catalysts are preferably used in which the ruthenium metal is supported on a carrier in amounts ranging from 0.05% to 10% by weight based on the entire weight of the catalyst.

The matters common to the inventions (8) to (11) are described below.

The carrier includes silica, alumina, silica alumina, zeolite, active carbon, titania, magnesia, and the like inorganic compounds, but is not limited thereto.

According to the invention (8), the amount of the metal supported by the carrier ranges preferably from 0.05% to 20% by weight, more preferably from 0.05% to 10% by weight, still more preferably from 2% to 10% by weight based on the entire weight of the catalyst. With the supported catalyst metal in an amount less than 0.05% by weight, the reaction time is longer, whereas with the supported catalyst metal of more than 20% by weight, the portion of the metal not participating in the isomerization is larger, disadvantageously.

The supported metal catalyst is used in an amount preferably from 0.01 to 50 parts by weight, more preferably from 1 to 30 parts by weight based on 100 parts by weight of the maleate. With the supported metal catalyst in an amount less than 0.01% by weight, the reaction time is longer, whereas with the supported metal catalyst in an amount of more than 50% by weight, the portion of the metal not participating in the isomerization is larger, disadvantageously.

The catalyst in the present invention may be used singly or in combination of two or more thereof.

The temperature of the isomerization reaction in the present invention ranges from 30°C to 200°C, preferably from 80°C to 180°C, more preferably from 120°C to 160°C. At the reaction temperature lower than 30°C, the reaction is slow, whereas at the reaction temperature higher than 200°C, side reactions of the ester or other functional group such as urethane proceed disadvantageously.

The isomerization reaction employing the catalyst supported by a carrier as described in the inventions (8) to (11) may be conducted in a solvent. The kind of the solvent is not limited, and specific examples of the solvent are shown later. The solvent may be a mixture of two or more solvents. In particular, presence of an alcohol can improve the catalyst activity.

For an oligomer having a higher molecular weight or a highly polymerizable compound which cannot readily be purified, the supported metal catalyst is especially useful for the production of a fumarate, since the supported metal catalyst can readily be separated by filtration or a like simple operation.

In the isomerization reaction of the present invention, for isomerization of the double bond of a maleic acid residue in highly polymerizable compound like an unsaturated polyester, a known polymerization inhibitor may be added.

The reaction pressure is not limited: the reaction may be conducted under a reduced pressure, under an atmospheric pressure, or under a high pressure. The atmosphere of the reaction system is inert gas such as nitrogen, helium, and argon. Preferably the reaction is conducted under a stream of inexpensive nitrogen. In isomerization by addition of a polymerization inhibitor, introduction of a very small amount of oxygen can be effective in polymerization inhibition for some kinds of the polymerization inhibitors, like a phenol type inhibitor.

Next, the present invention (12) is explained below in detail. The invention (12) relates to a method for producing a fumarate containing a 1-propenyloxy group, comprising isomerizing a maleate containing a 2-propenyloxy group in the molecule thereof, wherein the 2-propenyloxy group is isomerized to the 1-propenyloxy group and simultaneously the maleate is isomerized to a fumarate by use of an isomerization catalyst containing a Group VIII element.

The fumarates containing a 1-propenyloxy group are radical-polymerizable monomers. The fumarate is preferably produced by synthesizing a monomer containing a 2-propenyloxy group and a maleic acid residue, and isomerizing the 2-propyloxy group to a 1-propenyloxy group and the maleic acid residue to a fumaric acid residue simultaneously in one step under mild conditions. The inventors of the present invention show that the above process can be conducted by use of a catalyst containing a Group VIII element as the invention (12). The catalyst employed in the invention (12) is the same isomerization catalyst as that employed in the invention (1) containing a Group VIII element.

The present invention (13) relates to a method for producing a fumarate containing a 1-propenyloxy group through the above isomerization reaction by using a compound containing a Group VIII element as the isomerization catalyst

The type of the compound containing the Group VIII element employed in the invention (13) includes, similarly as in the invention (2), metals; inorganic salts such as sulfate, nitrate, and chlorides; organic acid salts such as acetates, and oxalates; and metal complexes having coordinated ligands. Of these types of compounds, particularly preferred are the metal complexes having a suitable ligand.

The ligand of the above metal complexes includes triphenylphosphine, carbonyl, hydrido, halogeno, aqua, cyclopentadiene, cyclooctadiene, acetylacetonato, and allyl ligands, but is not limited thereto.

The metal complex, as the catalyst, may be used as it is without modification. The metal complex may be formed during the reaction by adding the metal complex components, namely the metal, the ligand, and other component, separately to the reaction material to form the metal complex during the reaction. Otherwise, a compound formed by decomposition of the metal complex may be used as the catalyst.

The amount of the isomerization catalyst employed in the invention (13), similarly as in the invention (2), ranges preferably from 0.01 to 50.00 parts, more preferably from 0.10 to 30.00 parts by weight based on 100 parts by weight of the maleate. With the catalyst in an amount of less than 0.01 parts by weight, the isomerization reaction rate is lower, requiring a longer time for attaining a high isomerization ratio, which is disadvantagaeous industrially. With the catalyst in an amount of more than 50.00 parts by weight, the catalyst cost is higher, which is disadvantageous economically, although the reaction rate is higher.

The present inventions (14) to (16) are described in detail below. In the inventions (14) to (16), as the catalyst, a metal salt or a metal complex is employed which contains at least one of elements of ruthenium, rhodium, palladium, iridium, and platinum selected from the compounds containing a Group VIII element employed in the invention (13).

In the isomerization reaction of the maleate by use of a compound containing a Group VIII element as shown in the invention (13), the matters mentioned in the invention (3) are also true. That is, the use of a metal salt or a metal complex containing at least one element selected from the group of ruthenium, rhodium, palladium, iridium, and platinum gives high activity and forms less byproduct with a suitably selected ligand, in comparison with the use of a compound containing iron, cobalt, nickel, or a like element. Therefore, in industrial production of a fumarate containing a 1-propenyloxy group, use of ruthenium, rhodium, palladium, iridium, or platinum as the catalyst is advantageous economically in consideration of the overall production process.

Of the compounds containing ruthenium, rhodium, palladium, iridium, or platinum, particularly preferred are compounds containing ruthenium or rhodium; ruthenium salts, ruthenium complexes, rhodium salts, and rhodium complexes. Of these, the most suitable are $RuCl_2(PPh_3)_3$, $RhCl(CO)(PPh_3)_2$, and $RuClH(CO)(PPh_3)_3$.

The present invention (17) relates to the reaction of isomerizing a maleate containing a 2-propenyloxy group in the molecule, wherein a phosphine compound represented by the chemical formula $PR_3$ and/or a base is employed in addition to the isomerization catalyst in the reaction of isomerizing the 2-propoxy group to a 1-propenyloxy group and simultaneously isomerizing the maleate to a fumarate.

The invention (17) gives the effect of remarkable increase of the isomerization rate and improvement of the selectivity in the production of a fumarate containing a 1-propenyloxy group by use of a phosphine compound represented by the chemical formula $PR_3$ and/or a base in addition to the isomerization catalyst shown in the inventions (13) to (16).

The additive employed in the present invention (17) is a phosphine compound represented by the chemical formula $PR_3$ and/or a base. The substituent R of the phosphine compound $PR_3$ and a base are the same as described in the aforementioned invention (6).

The catalyst employed in the invention (17) may be a compound containing the same Group VIII element as in the invention (6).

The type of the compound containing the Group VIII element employed in the invention (17) is the same as that employed in the invention (6), including inorganic salts, organic acid salts, and metal complexes having a coordinated ligand. Of these particularly preferred are metal complexes having a suitable ligand.

The ligand of the metal complex includes triphenylphosphine, carbonyl, hydrido, halogeno, aqua, cyclopentadiene, cyclooctadiene, acetylacetonato, and allyl ligands, but is not limited thereto. The usage of such a metal complex as the catalyst is the same as that described in the invention (2).

The amount of the isomerization catalyst employed in the invention (17), similarly as in the invention (6), ranges preferably from 0.01 to 50.00 parts, more preferably from 0.10 to 30.00 parts by weight based on 100 parts by weight of the maleate. With the catalyst in an amount of less than 0.01 parts by weight, the isomerization reaction rate is lower, requiring a longer time for achieving a high isomerization ratio, which is disadvantagaeous industrially. With the catalyst in an amount of more than 50.00 parts by weight, the catalyst cost is higher, which is disadvantageous economically, although the reaction rate is higher.

The present invention (18) shows the optimum range of the amount of the phosphine compound and/or the base in the invention (17), wherein the phosphine compound represented by the chemical formula $PR_3$ and/or the base is employed in addition to the above isomerization catalyst. In the invention (17), the amount of the additive has an optimum range for the same reason described in the invention (6).

In the inventions (19) to (22) of the reaction of isomerizing a maleate containing 2-propenyloxy group in the molecule, the same description as in the aforementioned inventions (8) to (11) is applicable also to the simultaneous isomerization of a 2-propenyloxy group to a 1-propenyloxy group, and a maleate to a fumarate.

The invention (19) relates to a method for producing a fumarate containing a 1-propenyloxy group by use of one or more supported catalysts in which a Group VIII metal is supported on a carrier in an amount ranging from 0.05% to 10% by weight based on the total weight of the metal-supporting catalyst.

The invention (20) relates to a method for producing a fumarate containing a 1-propenyloxy group, wherein the Group VIII metal is at least one metal selected from the group of ruthenium, rhodium, palladium, iridium, and

platinum.

The invention (21) relates to a method for producing a fumarate containing a 1-propenyloxy group, wherein at least one of the Group VIII metal of the isomerization catalyst is ruthenium.

The invention (22) relates to a method for producing a fumarate containing a 1-propenyloxy group, wherein one or more supported catalysts are preferably used in which the ruthenium metal is supported on the carrier in an amount ranging from 0.05% to 10% by weight based on the entire weight of the catalyst.

The present inventions (1) to (22) are explained above respectively. Hereinafter, the matters common to the inventions (2) to (7) and to the inventions (13) to (18) are explained.

The reaction temperature of the isomerization in the present invention ranges from 0°C to 200°C, preferably from 50°C to 180°C, more preferably from 80°C to 160°C. At the reaction temperature lower than 0°C, the reaction rate is low, whereas at the reaction temperature higher than 200°C, the monomer tends to polymerize to lower the reaction yield.

The product by the isomerization reaction can be purified by a conventional purification method. The purification method conventionally includes distillation, extraction, recrystallization, gas chromatography, and dialysis, but is not limited thereto.

In the isomerization reaction according to the present invention, a known polymerization inhibitor may be added to the reaction system, especially in isomerization of a double bond of maleic acid residue in highly polymerizable compound such as an unsaturated polyester.

The reaction pressure is not specially limited; the reaction may be conducted under a reduced pressure, an atmospheric pressure, or a high pressure. The ambient atmosphere for the reaction is not limited, and may be a reducing atmosphere or an oxidizing atmosphere. The reaction may be conducted in an air atmosphere, or in an inert gas atmosphere such as atmosphere of nitrogen, helium, and argon, but is conducted preferably in an inexpensive nitrogen atmosphere. In the isomerization reaction by addition of a polymerization inhibitor, introduction of a very small amount of oxygen can be effective for some kinds of polymerization inhibitors like a phenol type inhibitor.

In the isomerization reaction, a liquid maleate as the source material may naturally be isomerized without a solvent. However, in the case where the maleate as the source material is solid, or where the maleate is liquid but does not dissolve the catalyst or other component or is not mixed uniformly, the reaction rate could be lower, or the reaction product could gel during the reaction disadvantageously.

Such a disadvantage can be offset by conducting the reaction in an appropriate solvent. The solvent is selected which dissolves well the maleate as the source material, the catalyst, the polymerization inhibitor, and other additive, and is not denatured or does not react with the source material during the reaction.

For conducting the isomerization reaction in a solvent, in the present invention, the solvent includes aromatic hydrocarbons such as benzene, toluene, and xylene; ethers such as diethyl ether, dimethoxyethane, methoxyethyl ether, tetrahydrofuran, and 1,4-dioxane; esters such as ethyl acetate, and butyl acetate; ketones such as acetone, and methyl ethyl ketone; and alcohols such as methanol, ethanol, 1-propanol, 2-propanol, 1-butanol, 2-butanol, t-butanol, and isobutanol; and water.

Naturally, the solvent may be used singly, or in combination of two or more thereof.

The amount of the solvent used therefor ranges preferably from 1 to 1000 parts by weight, more preferably from 10 to 200 parts by weight based on 100 parts by weight of the maleate, a source material. The solvent in an amount less than one part by weight is not sufficient for achieving the solvent effect, whereas the solvent in an amount of more than 1000 parts by weight is excessive to render the source material concentration lower to decrease the reaction rate disadvantageously.

The reactor vessel for the isomerization reaction of the present invention is not limited, and may be a glass vessel, glass-lined vessel, a stainless vessel, a Teflon vessel, or the like.

## EXAMPLES

The present invention is explained below specifically by reference to examples without limiting the invention thereto. The measurement was conducted with the apparatuses shown below.

$^1$H-NMR (60MHz proton NMR):

Apparatus: Hitachi R-1200 Model high-speed sweeping correlation NMR apparatus
Solvent: Deuterated chloroform
Internal standard: Tetramethylsilane (for chemical shift calculation)

GPC (Size exclusion chromatography):

Column: Shodex K-801; Thermostat: 40°C;
Detector: UV spectrometer (Waters 484 Tunable Absorbance Detector, detection wavelength: 254 nm)
Eluate: Chloroform 1 mL/min

The monomer selectivity, determined by GPC, is shown by the ratio of the total of the maleate and the fumarate (excluding the polymeric and oligomeric byproducts) to the source maleate.

EXAMPLE 1

A 10-mL glass reaction tube was charged with 2.00 g of dimethyl maleate, 2.00 g of ethanol, and 0.020 g of dichlorotris(triphenylphosphine)ruthenium (II) as the catalyst. The mixture was heated to 140°C, and was stirred at that temperature for three hours. Then, the solvent was removed from the reaction solution under a reduced pressure. The reaction mixture was analyzed by $^1$H-NMR and GPC. The dimethyl maleate was found to have been converted to dimethyl fumarate at a conversion ratio of 97%. The monomer selectivity was 99% according to GPC.

EXAMPLE 2

A 10-mL glass reaction tube was charged with 2.00 g of dimethyl maleate, 2.00 g of toluene, and 0.020 g of dichlorotris(triphenylphosphine)ruthenium (II) as the catalyst. The mixture was heated to 140°C, and was stirred at that temperature for three hours. Then, the solvent was removed from the reaction solution under a reduced pressure. The reaction mixture was analyzed by $^1$H-NMR and GPC. The dimethyl maleate was found to have been converted to dimethyl fumarate at a conversion ratio of 97%. The monomer selectivity was 98% according to GPC.

EXAMPLE 3

A 10-mL glass reaction tube was charged with 2.00 g of dimethyl maleate, 2.00 g of toluene, and 0.020 g of ruthenium trichloride as the catalyst. The mixture was heated to 140°C, and was stirred at that temperature for three hours. Then, the solvent was removed from the reaction solution under a reduced pressure. The reaction mixture was analyzed by $^1$H-NMR and GPC. The dimethyl maleate was found to have been converted to dimethyl fumarate at a conversion ratio of 73%. The monomer selectivity was 42% according to GPC.

EXAMPLE 4

A 10-mL glass reaction tube was charged with 2.00 g of bis(2-(2-propenyloxy)ethyl) maleate, 2.00 g of toluene, and 0.020 g of dichlorotris(triphenylphosphine)ruthenium (II) as the catalyst. The mixture was heated to 140°C, and was stirred at that temperature for three hours. Then, the solvent was removed from the reaction solution under a reduced pressure. The reaction mixture was analyzed by $^1$H-NMR and GPC. The maleic acid residue in the bis(2-(2-propenyloxy)ethyl) maleate was found to have been converted to a fumaric acid residue at a conversion ratio of 94%. The 2-propenyl group therein was found to have been converted to a 1-propenyl group at a conversion ratio of 92%. The monomer selectivity was 91% according to GPC.

EXAMPLE 5

A 10-mL glass reaction tube was charged with 2.00 g of bis(2-(2-propenyloxy)ethyl) maleate, 2.00 g of toluene, and 0.020 g of dichlorotetrakis(triphenylphosphine)ruthenium (II) as the catalyst. The mixture was heated to 140°C, and was stirred at that temperature for three hours. Then, the solvent was removed from the reaction solution under a reduced pressure. The reaction mixture was analyzed by $^1$H-NMR and GPC. The maleic acid residue in the bis (2-(2-propenyloxy)ethyl) maleate was found to have been converted to a fumaric acid residue at a conversion ratio of 95%. The 2-propenyl group therein was found to have been converted to a 1-propenyl group at a conversion ratio of 91%. The monomer selectivity was 92% according to GPC.

EXAMPLE 6

A 10-mL glass reaction tube was charged with 2.00 g of bis(2-(2-propenyloxy)ethyl) maleate, 2.00 g of toluene, and 0.020 g of carbonylchlorohydridotris(triphenylphosphine)ruthenium (II) as the catalyst. The mixture was heated to 140°C, and was stirred at that temperature for three hours. Then, the solvent was removed from the reaction solution under a reduced pressure. The reaction mixture was analyzed by $^1$H-NMR and GPC. The maleic acid residue in the bis(2-(2-propenyloxy)ethyl) maleate was found to have been converted to a fumaric acid residue at a conversion ratio

of 99%. The 2-propenyl group therein was found to have been converted to a 1-propenyl group at a conversion ratio of 85%. The monomer selectivity was 89% according to GPC.

EXAMPLE 7

A 10-mL glass reaction tube was charged with 2.00 g of bis(2-(2-propenyloxy)ethyl) maleate, 2.00 g of toluene, and 0.020 g of trirutheniumdodecacarbonyl as the catalyst. The mixture was heated to 140°C, and was stirred at that temperature for three hours. Then, the solvent was removed from the reaction solution under a reduced pressure. The reaction mixture was analyzed by $^1$H-NMR and GPC. The maleic acid residue in the bis(2-(2-propenyloxy)ethyl) maleate was found to have been converted to a fumaric acid residue at a conversion ratio of 85%. The 2-propenyl group therein was found to have been converted to a 1-propenyl group at a conversion ratio of 82%. The monomer selectivity was 87% according to GPC.

EXAMPLE 8

A 10-mL glass reaction tube was charged with 2.00 g of bis(2-(2-propenyloxy)ethyl) maleate, 2.00 g of toluene, and 0.020 g of chlorocarbonylbis(triphenylphosphine)rhodium (I) as the catalyst. The mixture was heated to 140°C, and was stirred at that temperature for three hours. Then, the solvent was removed from the reaction solution under a reduced pressure. The reaction mixture was analyzed by $^1$H NMR and GPC. The maleic acid residue in the bis (2-(2-propenyloxy)ethyl) maleate was found to have been converted to a fumaric acid residue at a conversion ratio of 79%. The 2-propenyl group therein was found to have been converted to a 1-propenyl group at a conversion ratio of 75%. The monomer selectivity was 92% according to GPC.

EXAMPLE 9

A 10-mL glass reaction tube was charged with 2.00 g of bis(2-(2-propenyloxy)ethyl) maleate, 2.00 g of toluene, 0.002 g of dichlorotris(triphenylphosphine)ruthenium (II) as the catalyst, and 0.02 g of triphenylphosphine as an additive. The mixture was heated to 140°C, and was stirred at that temperature for three hours. Then, the solvent was removed from the reaction solution under a reduced pressure. The reaction mixture was analyzed by $^1$H-NMR and GPC. The maleic acid residue in the bis(2-(2-propenyloxy)ethyl) maleate was found to have been converted to a fumaric acid residue at a conversion ratio of 89%. The 2-propenyl group therein was found to have been converted to a 1-propenyl group at a conversion ratio of 76%. The monomer selectivity was 96% according to GPC.

EXAMPLE 10

A 10-mL glass reaction tube was charged with 2.00 g of bis(2-(2-propenyloxy)ethyl) maleate, 2.00 g of toluene, 0.002 g of dichlorotris(triphenylphosphine)ruthenium (II) as the catalyst, and 0.02 g of morpholine as an additive. The mixture was heated to 140°C, and was stirred at that temperature for three hours. Then, the solvent was removed from the reaction solution under a reduced pressure. The reaction mixture was analyzed by $^1$H-NMR and GPC. The maleic acid residue in the bis(2-(2-propenyloxy)ethyl) maleate was found to have been converted to a fumaric acid residue at a conversion ratio of 69%. The 2-propenyl group therein was found to have been converted to a 1-propenyl group at a conversion ratio of 90%. The monomer selectivity was 98% according to GPC.

EXAMPLE 11

A 10-mL glass reaction tube was charged with 2.00 g of bis(2-(2-propenyloxy)ethyl) maleate, 2.00 g of toluene, 0.002 g of dichlorotris(triphenylphosphine)ruthenium (II) as the catalyst, and 0.02 g of triphenylphosphine and 0.02 g of morpholine as the additives. The mixture was heated to 140°C, and was stirred at that temperature for three hours. Then, the solvent was removed from the reaction solution under a reduced pressure. The reaction mixture was analyzed by $^1$H-NMR and GPC. The maleic acid residue in the bis(2-(2-propenyloxy)ethyl) maleate was found to have been converted to a fumaric acid residue at a conversion ratio of 86%. The 2-propenyl group therein was found to have been converted to a 1-propenyl group at a conversion ratio of 87%. The monomer selectivity was 97% according to GPC.

EXAMPLE 12

A 10-mL glass reaction tube was charged with 2.00 g of bis(2-(2-propenyloxy)ethyl) maleate, and 0.02 g of dichlorotris(triphenylphosphine)ruthenium (II) as the catalyst. The mixture was heated to 140°C, and was stirred at that temperature for three hours. The reaction mixture was analyzed by $^1$H-NMR and GPC. The maleic acid residue

in the bis(2-(2-propenyloxy)ethyl) maleate was found to have been converted to a fumaric acid residue at a conversion ratio of 68%. The 2-propenyl group therein was found to have been converted to a 1-propenyl group at a conversion ratio of 62%. The monomer selectivity was 45% according to GPC.

EXAMPLE 13

A 10-mL glass reaction tube was charged with 2.00 g of bis(2-(2-propenyloxy)ethyl) maleate, 2.00 g of toluene, and 0.002 g of dichlorotris(triphenylphosphine)ruthenium (II) as the catalyst. The mixture was heated to 140°C, and was stirred at that temperature for three hours. Then, the solvent was removed from the reaction solution under a reduced pressure. The reaction mixture was analyzed by $^1$H-NMR and GPC. The maleic acid residue in the bis(2-(2-propenyloxy)ethyl) maleate was found to have been converted to a fumaric acid residue at a conversion ratio of 35%. The 2-propenyl group therein was found to have been converted to a 1-propenyl group at a conversion ratio of 28%. The monomer selectivity was 92% according to GPC.

EXAMPLE 14

A 10-mL glass reaction tube was charged with 2.00 g of dimethyl maleate, 2.00 g of toluene, and 0.002 g of dichlorotris(triphenylphosphine)ruthenium (II) as the catalyst. The mixture was heated to 140°C, and was stirred at that temperature for three hours. Then, the solvent was removed from the reaction solution under a reduced pressure. The reaction mixture was analyzed by $^1$H-NMR and GPC. The dimethyl maleate was found to have been converted to dimethyl fumarate at a conversion ratio of 36%. The monomer selectivity was 98% according to GPC.

EXAMPLE 15

A 10-mL glass reaction tube was charged with 2.00 g of dimethyl maleate, 2.00 g of toluene, 0.002 g of dichlorotris(triphenylphosphine)ruthenium (II) as the catalyst, and 0.02 g of morpholine as an additive. The mixture was heated to 140°C, and was stirred at that temperature for three hours. Then, the solvent was removed from the reaction solution under a reduced pressure. The reaction mixture was analyzed by $^1$H-NMR and GPC. The dimethyl maleate was found to have been converted to dimethyl fumarate at a conversion ratio of 71%. The monomer selectivity was 97% according to GPC.

COMPARATIVE EXAMPLE 1

A 10-mL glass reaction tube was charged with 2.00 g of bis(2-(2-propenyloxy)ethyl) maleate, 2.00 g of toluene, and 0.020 g of 36% concentrated hydrochloric acid as an additive. The mixture was heated to 140°C, and was stirred at that temperature for three hours. Then, the solvent was removed from the reaction solution under a reduced pressure. The reaction mixture was analyzed by $^1$H-NMR and GPC. The maleic acid residue in the bis(2-(2-propenyloxy)ethyl) maleate was found to have been converted to a fumaric acid residue at a conversion ratio of 23%. The conversion ratio of the 2-propenyl group therein was found to be 0%. The monomer selectivity was 52% according to GPC.

COMPARATIVE EXAMPLE 2

A 10-mL glass reaction tube was charged with 2.00 g of bis(2-(2-propenyloxy)ethyl) maleate, 2.00 g of toluene, and 0.02 g of triphenylphosphine as an additive. The mixture was heated to 140°C, and was stirred at that temperature for three hours. Then, the solvent was removed from the reaction solution under a reduced pressure. The reaction mixture was analyzed by $^1$H-NMR and GPC. The maleic acid residue in the bis(2-(2-propenyloxy)ethyl) maleate was found to have been converted to a fumaric acid residue at a conversion ratio of 13%. The conversion ratio of the 2-propenyl group therein was found to be 0%. The monomer selectivity was 82% according to GPC.

COMPARATIVE EXAMPLE 3

A 10-mL glass reaction tube was charged with 2.00 g of bis(2-(2-propenyloxy)ethyl) maleate, 2.00 g of toluene, and 0.02 g of morpholine as an additive. The mixture was heated to 140°C, and was stirred at that temperature for three hours. Then, the solvent was removed from the reaction solution under a reduced pressure. The reaction mixture was analyzed by $^1$H-NMR and GPC. The maleic acid residue in the bis(2-(2-propenyloxy)ethyl) maleate was found to have been converted to a fumaric acid residue at a conversion ratio of 7%. The dimethyl maleate was found to have been converted to dimethyl fumarate at a conversion ratio of 3%. The monomer selectivity was 85% according to GPC.
Table 1 summarizes the results of Examples 1-15 and Comparative Examples 1-3.

Table 1

| | Source material | | Solvent | | Catalyst | | Additive | | Isomerization % | | Selectivity |
|---|---|---|---|---|---|---|---|---|---|---|---|
| | Compound | Grams | Solvent | Grams | Compound | Grams | Compound | Grams | Fumaric | 1-Propenyl | Monomer % |
| Example | | | | | | | | | | | |
| 1 | DMM | 2.00 | Ethanol | 2.00 | $RuCl_2(PPh_3)_3$ | 0.020 | --- | --- | 97 | --- | 99 |
| 2 | DMM | 2.00 | Toluene | 2.00 | $RuCl_2(PPh_3)_3$ | 0.020 | --- | --- | 97 | --- | 98 |
| 3 | DMM | 2.00 | Toluene | 2.00 | $RuCl_3$ | 0.020 | --- | --- | 73 | --- | 42 |
| 4 | PEM | 2.00 | Toluene | 2.00 | $RuCl_2(PPh_3)_3$ | 0.020 | --- | --- | 94 | 92 | 91 |
| 5 | PEM | 2.00 | Toluene | 2.00 | $RuCl_2(PPh_3)_4$ | 0.020 | --- | --- | 95 | 91 | 92 |
| 6 | PEM | 2.00 | Toluene | 2.00 | $RuClH(CO)(PPh_3)_3$ | 0.020 | --- | --- | 99 | 85 | 89 |
| 7 | PEM | 2.00 | Toluene | 2.00 | $Ru_3(CO)_{12}$ | 0.020 | --- | --- | 85 | 82 | 87 |
| 8 | PEM | 2.00 | Toluene | 2.00 | $RhCl(CO)(PPh_3)_2$ | 0.020 | --- | --- | 79 | 75 | 92 |
| 9 | PEM | 2.00 | Toluene | 2.00 | $RuCl_2(PPh_3)_3$ | 0.002 | TPP | 0.02 | 89 | 76 | 96 |
| 10 | PEM | 2.00 | Toluene | 2.00 | $RuCl_2(PPh_3)_3$ | 0.002 | MP | 0.02 | 69 | 90 | 98 |
| 11 | PEM | 2.00 | Toluene | 2.00 | $RuCl_2(PPh_3)_3$ | 0.002 | TPP<br>MP | 0.02<br>0.02 | 86 | 87 | 97 |
| 12 | PEM | 2.00 | --- | --- | $RuCl_2(PPh_3)_3$ | 0.020 | --- | --- | 68 | 62 | 45 |
| 13 | PEM | 2.00 | Toluene | 2.00 | $RuCl_2(PPh_3)_3$ | 0.002 | --- | --- | 35 | 28 | 92 |
| 14 | DMM | 2.00 | Toluene | 2.00 | $RuCl_2(PPh_3)_3$ | 0.002 | --- | --- | 36 | --- | 98 |
| 15 | DMM | 2.00 | Toluene | 2.00 | $RuCl_2(PPh_3)_3$ | 0.002 | MP | 0.02 | 91 | --- | 97 |
| Comparative Example | | | | | | | | | | | |
| 1 | PEM | 2.00 | Toluene | 2.00 | --- | 0.020 | 36%HCl | --- | 23 | 0 | 52 |
| 2 | PEM | 2.00 | Toluene | 2.00 | --- | --- | TPP | 0.02 | 13 | 0 | 82 |
| 3 | PEM | 2.00 | Toluene | 2.00 | --- | --- | MP | 0.02 | 7 | 3 | 85 |

Reaction temperture: 140℃, Reaction time: 3 hours

DMM: Dimethyl maleate, PEM: Bis(2-(2-propenyloxy)ethyl) maleate,
TPP: Triphenylphosphine, MP: Morpholine

EXAMPLE 16

A 20-mL autoclave was charged with 2.06 g of dimethyl maleate, 435 mg of Ru/active carbon (metal content 5% by weight), and 4 mL of ethanol. The mixture was heated to 150°C, and was stirred at that temperature for two hours. Thereby, a white solid matter was deposited. The reaction mixture was dissolved by addition of methanol. The catalyst was removed by filtration. From the resulting filtrate, the solvent was evaporated under a reduced pressure to obtain 1.96 g of a white solid matter. The isomerization ratio to dimethyl fumarate was confirmed to be 100% by NMR analysis.

EXAMPLE 17

A 20-mL autoclave was charged with 2.07 g of dimethyl maleate, 437 mg of Ru/active carbon (metal content 5% by weight), and 4 mL of 1-propanol. The mixture was heated to 150°C, and was stirred at that temperature for two hours. Thereby, a white solid matter was deposited. The reaction mixture was dissolved by addition of methanol. The catalyst was removed by filtration. From the resulting filtrate, the solvent was evaporated under a reduced pressure to obtain 1.95 g of a white solid matter. The isomerization ratio to dimethyl fumarate was confirmed to be 100% by NMR analysis.

EXAMPLE 18

A 20-mL autoclave was charged with 2.01 g of dimethyl maleate, 423 mg of Ru/active carbon (metal content 5% by weight), and 4 mL of 2-propanol. The mixture was heated to 150°C, and was stirred at that temperature for two hours. Thereby, a white solid matter was deposited. The reaction mixture was dissolved by addition of methanol. The catalyst was removed by filtration. From the resulting filtrate, the solvent was evaporated under a reduced pressure to obtain 1.93 g of a white solid matter. The isomerization ratio to dimethyl fumarate was confirmed to be 100% by NMR analysis.

EXAMPLE 19

A 20-mL autoclave was charged with 2.02 g of dimethyl maleate, 427 mg of Ru/active carbon (metal content 5% by weight), and 4 mL of 1-butanol. The mixture was heated to 150°C, and was stirred at that temperature for two hours. Thereby, a white solid matter was deposited. The reaction mixture was dissolved by addition of methanol. The catalyst was removed by filtration. From the resulting filtrate, the solvent was evaporated under a reduced pressure to obtain 1.94 g of a white solid matter. The isomerization ratio to dimethyl fumarate was confirmed to be 100% by NMR analysis.

EXAMPLE 20

A 20-mL autoclave was charged with 2.07 g of dimethyl maleate, and 436 mg of Ru/active carbon (metal content 5% by weight). After replacing the inside atmosphere with nitrogen, the mixture was heated to 150°C, and was stirred at that temperature for two hours. Thereby, a white solid matter was deposited. The reaction mixture was dissolved by addition of methanol. The catalyst was removed by filtration. From the resulting filtrate, the solvent was evaporated under a reduced pressure to obtain 1.99 g of a white solid matter. The isomerization ratio of the dimethyl maleate to dimethyl fumarate was confirmed to be 74% by NMR analysis.

EXAMPLE 21

A one-liter autoclave was charged with 300.0 g of bis(2-(2-propenyloxy)ethyl) maleate, 6.4 g of Ru/active carbon (metal content 5% by weight), 30 mg of tetrakis[methylene-3-(3,5-di-t-butyl-4-hydroxyphenyl) propionate]methane (trade name: IRGANOX1010, produced by Ciba-Geigy Japan Ltd.) as a polymerization inhibitor, and 300 mL of 2-propanol. After replacing the inside atmosphere with nitrogen, the mixture was heated to 150°C, and was stirred at that temperature for two hours. After cooling, the catalyst was removed by filtration. From the resulting filtrate, the solvent was evaporated under a reduced pressure to obtain 270.1 g of a yellow liquid. The isomerization ratio of the maleoyl double bond to a fumaroyl double bond was confirmed to be 94% by NMR analysis.

EXAMPLE 22

A 300-mL autoclave was charged with 62.4 g of an oligomer produced by condensation of dimethyl maleate

with an adduct of bisphenol A and 2-mole ethylene oxide in a mol ratio of 3:1 by transesterification, 62 mL of toluene, 62 mL of 1-propanol, and 6.8 g of Ru/active carbon (metal content 5% by weight). After replacing the inside atmosphere with nitrogen, the mixture was heated to 150°C, and was stirred at that temperature for 10 hours. After cooling, the catalyst was removed by filtration. From the resulting filtrate, the solvent was evaporated under a reduced pressure to obtain 59.2 g of a yellow viscous liquid. The isomerization ratio of the maleoyl double bond to a fumaroyl double bond was confirmed to be 99% by NMR analysis.

EXAMPLE 23

A 20-mL autoclave was charged with 5.12 g of bis(2-(2-propenyloxy)ethyl) maleate, and 565.1 mg of Pd-$Al_2O_3$ (metal content: 5% by weight). After replacing the inside atmosphere with nitrogen, the mixture was heated to 150°C, and was stirred at that temperature for 10 hours. After cooling, the catalyst was removed by filtration to obtain 4.8 g of a yellow liquid. The isomerization ratio of the maleoyl double bond to a fumaroyl double bond was confirmed to be 75% by NMR analysis.

EXAMPLE 24

A 20 mL autoclave was charged with 2.02 g of bis(2-(2-propenyloxy)ethyl) maleate, and 213.8 mg of Ru/active carbon (metal content: 5% by weight). After replacing the inside atmosphere with nitrogen, the mixture was heated to 150°C, and was stirred at that temperature for 5 hours. After cooling, the catalyst was removed by filtration to obtain 2.0 g of a yellow liquid. The isomerization ratio of the maleoyl double bond to a fumaroyl double bond was confirmed to be 84% by NMR analysis.

EXAMPLE 25

A 20-mL autoclave was charged with 1.97 g of bis(2-(2-propenyloxy)ethyl) maleate, 210.4 mg of Ru/active carbon (metal content: 5% by weight), and 4 mL of n-butanol. After replacing the inside atmosphere with nitrogen, the mixture was heated to 150°C, and was stirred at that temperature for two hours. After cooling, the catalyst was removed by filtration. The solvent was evaporated under a reduced pressure to obtain 1.90 g of a yellow liquid. The isomerization ratio of the maleoyl double bond to a fumaroyl double bond was confirmed to be 100% by NMR analysis.

EXAMPLE 26

A 20-mL autoclave was charged with 2.06 g of bis(2-(2-propenyloxy)ethyl) maleate, and 44.2 mg of Rh-$Al_2O_3$ (metal content: 5% by weight). After replacing the inside atmosphere with nitrogen, the mixture was heated to 150°C, and was stirred at that temperature for 10 hours. After cooling, the catalyst was removed by filtration to obtain 1.72 g of a yellow liquid. The isomerization ratio of the maleoyl double bond to a fumaroyl double bond was confirmed to be 80% by NMR analysis.

Effects of the Invention

The method of the present invention produces a fumarate in a high yield at a high selectivity by isomerizing a maleate in the presence of a catalyst. This method is particularly useful in the fields of resin source materials, plasticizers, and the like. Further, the method of the present invention enables maleates containing a 2-propenyloxy group to isomerize its 2-propyloxy group to a 1-propenyl group, and the maleates to fumarates in one step effectively. Furthermore, the high-purity fumarate containing no catalyst residue can be obtained by use of a heterogeneous catalyst by a simple separation operation without corrosion.

## Claims

1. A method for producing a fumarate by isomerizing a maleate, comprising using an isomerization catalyst containing a Group VIII element.

2. The method for producing a fumarate according to claim 1, wherein the isomerization catalyst is a compound containing a Group VIII element.

3. The method for producing a fumarate according to claim 2, wherein the isomerization catalyst is a metal salt or a

metal complex containing at least one element selected from the group consisting of ruthenium, rhodium, palladium, iridium, and platinum.

**4.** The method for producing a fumarate according to claim 3, wherein the isomerization catalyst is a ruthenium salt, a ruthenium complex, a rhodium salt, or a rhodium complex.

**5.** The method for producing a fumarate according to claim 4, wherein the isomerization catalyst is $RuCl_2(PPh_3)_3$, $RhCl(CO)(PPh_3)_2$, or $RuClH(CO)(PPh_3)_3$.

**6.** The method for producing a fumarate according to claim 2, wherein a phosphine compound represented by a chemical formula $PR_3$ (R denoting independently an alkyl, a cycloalkyl, or a phenyl), and/or a base is employed in addition to the above isomerization catalyst.

**7.** The method for producing a fumarate according to claim 6, wherein the amount of the added phosphine compound ranges from 0.01 to 50.00 parts by weight based on 100 parts by weight of the maleate, and/or the amount of the added base ranges from 0.01 to 50.00 parts by weight based on 100 parts by weight of the maleate.

**8.** The method for producing a fumarate according to claim 1, wherein the isomerization catalyst is one or more kinds of supported catalyst in which a Group VIII metal is supported on a carrier in an amount ranging from 0.05% to 10% by weight based on the entire weight of the catalyst.

**9.** The method for producing a fumarate according to claim 8, wherein the Group VIII metal is at least one metal selected from the group consisting of ruthenium, rhodium, palladium, iridium, and platinum.

**10.** The method for producing a fumarate according to claim 9, wherein at least one metal of the Group VIII metals of the isomerization catalyst is ruthenium.

**11.** The method for producing a fumarate according to claim 10, wherein one or more supported catalysts are used in which the ruthenium metal is supported on the carrier in an amount ranging from 0.05% to 10% by weight based on the entire weight of the catalyst.

**12.** A method for producing a fumarate containing a 1-propenyloxy group, comprising isomerizing a maleate containing a 2-propenyloxy group in the molecule thereof to the fumarate containing the 1-propenyloxy group by use of an isomerization. catalyst containing a Group VIII element.

**13.** The method for producing a fumarate containing a 1-propenyloxy group according to claim 12, wherein the isomerization catalyst is a compound containing a Group VIII element.

**14.** The method for producing a fumarate containing a 1- propenyloxy group according to claim 13, wherein the isomerization catalyst is a metal salt or a metal complex containing at least one element selected from the group consisting of ruthenium, rhodium, palladium, iridium, and platinum.

**15.** The method for producing a fumarate containing a 1-propenyloxy group according to claim 14, wherein the isomerization catalyst is a ruthenium salt, a ruthenium complex, a rhodium salt, or a rhodium complex.

**16.** The method for producing a fumarate containing a 1-propenyloxy group according to claim 15, wherein the isomerization catalyst is $RuCl_2(PPh_3)_3$, $RhCl(CO)(PPh_3)_2$, or $RuClH(CO)(PPh_3)_3$.

**17.** The method for producing a fumarate containing a 1-propenyloxy group according to claim 13, wherein a phosphine compound represented by the chemical formula $PR_3$ (R denoting independently an alkyl, a cycloalkyl, or a phenyl), and/or a base is employed in addition to the above isomerization catalyst.

**18.** The method for producing a fumarate containing a 1-propenyloxy group according to claim 17, wherein the amount of the added phosphine compound ranges from 0.01 to 50.00 parts by weight based on 100 parts by weight of the maleate, and/or the amount of the added base ranges from 0.01 to 50.00 parts by weight based on 100 parts by weight of the maleate.

**19.** The method for producing a fumarate containing a 1-propenyloxy group according to claim 12, wherein the isomerization catalyst is one or more kinds of supported catalyst in which the Group VIII metal is supported on a carrier in an amount ranging from 0.05% to 10% by weight based on the entire weight of the catalyst.

**20.** The method for producing a fumarate containing a 1-propenyloxy group according to claim 19, wherein the Group VIII metal is at least one metal selected from the group consisting of ruthenium, rhodium, palladium, iridium, and platinum.

**21.** The method for producing a fumarate containing a 1-propenyloxy group according to claim 20, wherein at least one metal of the Group VIII metals of the isomerization catalyst is ruthenium.

**22.** The method for producing a fumarate containing a 1-propenyloxy group according to claim 21, wherein one or more supported catalysts are used in which ruthenium metal is supported on the carrier in an amount ranging from 0.05% to 10% by weight based on the entire weight of the catalyst.

# INTERNATIONAL SEARCH REPORT

International application No.
PCT/JP01/01028

A. CLASSIFICATION OF SUBJECT MATTER
Int.Cl$^7$ C07C67/333, 69/60, C08G18/83, 63/91//C07B61/00, B01J31/24, 23/46, 27/13

According to International Patent Classification (IPC) or to both national classification and IPC

B. FIELDS SEARCHED

Minimum documentation searched (classification system followed by classification symbols)
Int.Cl$^7$ C07C67/333, 69/60, 51/353, 57/15, C08G18/83, 63/91//C07B61/00, B01J31/24, 23/46, 27/13

Documentation searched other than minimum documentation to the extent that such documents are included in the fields searched

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| P,Y | JP, 2000-169423, A (Mitsubishi Chemical Corporation), 20 June, 2000 (20.06.00) (Family: none) | 1 |
| Y | JP, 8-24664, A (Nippon Shokubai Co., Ltd.), 30 January, 1996 (30.01.96) (Family: none) | 1 |
| Y | JP, 5-279292, A (Kawasaki Kasei Chem. Ltd.), 26 October, 1993 (26.10.93) (Family: none) | 1 |
| Y | JP, 63-68529, A (Huels AG), 28 March, 1988 (28.03.88) & EP, 262310, A1 & DE, 3629512, A1 | 1-5,8,9, 12-16,19,20 |
| Y | JP, 51-29401, A (Kuraray Co., Ltd.), 12 March, 1976 (12.03.76) (Family: none) | 1-4,8-11, 12-15,19-22 |
| X<br>Y | JP, 50-17044, B1 (Agency of Industrial Science and Technology), 18 June, 1975 (18.06.75) (Family: none) | 1-3,8<br>4,5,9-11, 12-16,19-22 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
28 March, 2001 (28.03.01)

Date of mailing of the international search report
10 April, 2001 (10.04.01)

Name and mailing address of the ISA/
Japanese Patent Office

Facsimile No.

Authorized officer

Telephone No.

Form PCT/ISA/210 (second sheet) (July 1992)

**INTERNATIONAL SEARCH REPORT**

International application No.
PCT/JP01/01028

C (Continuation). DOCUMENTS CONSIDERED TO BE RELEVANT

| Category* | Citation of document, with indication, where appropriate, of the relevant passages | Relevant to claim No. |
|---|---|---|
| Y | EP, 957079, A1 (Showa Denko Kabushiki Kaisha), 17 November, 1999 (17.11.99) & WO, 99/02482, A1 & AU, 9881285, A | 12-16,19-22 |
| Y | JP, 11-255691, A (Sumitomo Chemical Company, Limited), 21 September, 1999 (21.09.99) (Family: none) | 12-16,19-22 |
| Y | JP, 5-17383, A (Mitsubishi Petrochemical Co., Ltd.), 26 January, 1993 (26.01.93) (Family: none) | 12-16,19-22 |
| Y | JP, 5-17382, A (Mitsubishi Petrochemical Co., Ltd.), 26 January, 1993 (26.01.93) (Family: none) | 12-16,19-22 |
| A | US, 4868267, A (Owens-Corning Fiberglas), 19 September, 1989 (19.09.89) & EP, 348491, A1 & JP, 2-502925, A | 1-22 |

Form PCT/ISA/210 (continuation of second sheet) (July 1992)